# EUROPÄISCHE PATENTSCHRIFT

(11) **EP 0 544 144 B1**
(45) Veröffentlichungstag und Bekanntmachung des Hinweises auf die Patenterteilung: **14.06.1995**
(21) Anmeldenummer: 92119226.6
(22) Anmeldetag: 11.11.1992
(51) Int. Cl.: A61K 9/20

(54) **Feste pharmazeutische Retardform**
Solid pharmaceutical retard form
Forme pharmaceutique retard solide

(30) Priorität: 23.11.1991 DE 4138513
(43) Veröffentlichungstag der Anmeldung: 02.06.1993
(73) Patentinhaber: BASF Aktiengesellschaft, 67063 Ludwigshafen (DE)
(72) Erfinder: Mueller, Winfried, Dr., W-6800 Mannheim 1 (DE); Spengler, Reinhard, Dr., 6701 Maxdorf (DE); Grabowski, Sven, Dr., W-6700 Ludwigshafen (DE); Sanner, Axel, Dr., W-6710 Frankenthal (DE)

(56) Entgegenhaltungen:
- EP-A- 0 142 877
- EP-A- 0 240 904
- EP-A- 0 240 906
- EP-A- 0 358 107

## Beschreibung

Die Erfindung betrifft eine durch Schmelzextrusion und Formgebung hergestellte feste pharmazeutische Retardform, bei der der Wirkstoff in eine Mischung von mindestens einem bestimmten wasserunlöslichen und mindestens einem bestimmten wasserlöslichen Polymeren eingebettet ist. Die Formgebung erfolgt kontinuierlich, vorzugsweise durch Heißabschlag.

US 3 432 592 beschreibt den Spritzguß von wirkstoffhaltigen Polymerschmelzen. Die dort verwendeten Polymeren sollen ausdrücklich im Verdauungstrakt löslich oder mindestens teilweise löslich sein, damit die Freisetzung des Wirkstoffs gewährleistet ist. Solche teilweise löslichen Tabletten sind jedoch mechanisch empfindlich, so daß sie durch die Peristaltik im Verdauungstrakt beschädigt werden können und eine gleichmäßige Wirkstofffreisetzung nicht mehr gewährleistet ist. Als lösliche Polymere sind nur folgende belegt: ein im Pharmabereich nicht übliches, komplexes Polymeres aus einem Aminodiol und einem Epoxid sowie der bekanntlich thermoplastisch schwierig zu verarbeitende Polyvinylalkohol oder ein partiell verseiftes Copolymeres von Vinylacetat und Crotonsäure, das nur bei höheren pH-Werten löslich ist. Hinzu kommen bei diesem Verfahren die Nachteile des Spritzgusses wie lange Verweilzeit bei hoher Temperatur und hohe Materialverluste durch die Angußkanäle, deren Inhalt keiner Wiederverwendung zugeführt werden darf. Außerdem sind die Formkosten relativ zur Produktionsgeschwindigkeit außerordentlich hoch.

Das Extrudieren von wirkstoffhaltigen Schmelzen wasserlöslicher Polymerer ist aus EP-A 240 904 und EP-A 240 906 bekannt. Es hat sich jedoch gezeigt, daß die so hergestellten Produkte in vielen Fällen wenig lagerbeständig sind, da der Retardeffekt mit der Zeit abnimmt.

Der Erfindung lag die Aufgabe zugrunde, eine durch Extrusion und kontinuierliche Formgebung hergestellte feste Retardform ohne diese Nachteile zu entwickeln. Der Vorteil des Extrusionsverfahrens gegenüber anderen Techniken wie Granulieren und Tablettieren besteht in der einfachen Technologie, der Vermeidung von Lösungsmitteln, minimierter Anzahl und Menge an Hilfsstoffen, der Möglichkeit der Herstellung von festen Lösungen, der Vermeidung aufwendiger Mischprozesse und vor allem in der Vermeidung von Möglichkeiten zur Entmischung der Komponenten, mit anderen Worten in einer zuverlässig absolut gleichmäßigen Zusammensetzung der einzelnen Retardformen während der gesamten Produktion. Hinzu kommen die Vorteile eines kontinuierlichen Verfahrensablaufes mit hohem Durchsatz bei geringen Materialverlusten.

Überraschenderweise hat sich gezeigt, daß durch Extrusion und kontinuierliche Formgebung durch Kalandrierung oder Kalt- oder Heißabschlag hergestellte Retardformen aus einer wirkstoffhaltigen Mischung mindestens eines wasserunlöslichen und mindestens eines wasserlöslichen Polymeren gemäß Anspruch 1 auch bei mechanischer Beanspruchung stabil sind, d.h., daß nach dem Herauslösen der löslichen Anteile ein stabiles Gerüst aus unlöslichem Polymer erhalten bleibt, selbst wenn dessen Anteil an der gesamten Retardform nur 6 Gew.-% beträgt. Dadurch wird bei der in-vitro-Prüfung die Freisetzungsgeschwindigkeit unabhängig von der Paddle-Drehzahl. Außerdem sind sie - was ebenfalls nicht vorhersehbar war - lagerbeständig, der Retardeffekt nimmt nicht mit der Zeit ab. Die erfindungsgemäßen Retardformen sind somit allen in ähnlich einfacher Weise durch Extrusion und Spritzguß hergestellten bisher bekannten Retardformen überlegen.

Erfindungsgegenstand ist daher eine feste pharmazeutische Retardform, hergestellt durch Schmelzextrusion bei 50 bis 200°C und kontinuierliche Formgebung einer Mischung von 0,1 bis 70 Gew.-%, bezogen auf die fertige Retardform, eines pharmazeutischen Wirkstoffs mit einer Polymerschmelze folgender Zusammensetzung:
a) mindestens 6 Gew.-%, bezogen auf die gesamte Retardform, mindestens eines wasserunlöslichen Poly(meth)acrylates mit einer Glastemperatur Tg im Bereich -60 bis 180°C,
b) eine wasserlösliche Hydroxyalkylcellulose oder Hydroxyalkylmethylcellulose mit 2 oder 3 C-Atomen im Hydroxyalkylrest oder ein N-Vinylpyrrolidonpolymerisat mit 0 bis 50 Gew.% Vinylacetat oder eine Mischung von beiden im Mischungsverhältnis a):b) = 5:95 bis 95:5, vorzugsweise 15:85 bis 85:15, und
c) 0 bis 30 Gew.%, bezogen auf die fertige Retardform, eines oder mehrerer der üblichen Pharmahilfsstoffe.

Feste pharmazeutische Retardformen sind z.B. Tabletten, Drageekerne, Pellets, Granulate und Zäpfchen mit verzögerter Wirkstofffreisetzung. Pulver und Kapseln sind nicht gemeint.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 70, vorzugsweise von 0,5 bis 60 Gew.-% liegen.

Als wasserunlösliches Polymer a) kommen nur solche in Betracht, die keine physiologisch bedenklichen Monomeren enthalten und auch beim Extrudieren keine abspalten, und die zäh und elastisch genug sind, daß die daraus hergestellten festen pharmazeutischen Retardformen im Verdauungstrakt nicht beschädigt werden. Es wurde gefunden, daß diese Bedingungen in hervorragender Weise erfüllt werden durch wasserunlösliche Polyacrylate und Polymethacrylate mit Glastemperaturen Tg im Bereich von -60 bis 180°C, vorzugsweise 0 bis 150°C. Dies sind vor allem Polyacrylester von Alkoholen mit 1 bis 8 Kohlenstoffatomen und Polymethacrylester von Alkoholen mit 1 bis 4 Kohlenstoffatomen sowie deren Mischungen, vorzugsweise Copolymerisate von Acrylaten und/oder Methacrylaten von Alkoholen mit 1 bis 4 Kohlenstoffatomen untereinander und mit 0 bis 20 Mol.% an Acrylaten und/oder Methacrylaten, die in ihrer C₁- bis C₄-Alkoholkomponente eine quaternäre Ammoniumgruppe enthalten. Diese bewirken eine gewisse Quellbarkeit des Polymeren, die sich auf die Wirkstofffreisetzung günstig auswirken kann. Als Beispiele seien Copolymerisate aus Ethylacrylat-Methylmethacrylat-Trimethylammonioethylmethacrylat-chlorid im Molverhältnis 1:2:0,1 bis 1:2:0,2 genannt.

Die wasserlösliche Polymerkomponente b) besteht aus Hydroxyalkylcellulose oder Hydroxyalkylmethylcellulose mit 2 oder 3 C-Atomen im Hydroxyalkylrest und einem Hydroxyalkylierungsgrad von 0,5 bis 70 und/oder einem Methoxylierungsgrad von 0 bis 35 % oder einem Vinylpyrrolidonhomo- oder copolymerisat mit bis zu 50 Gew.% Vinylacetat oder einer Mischung von beiden.

"Wasserlöslich" bzw. "wasserunlöslich" heißt, daß sich im Liter Wasser von 20°C mindestens 10, vorzugsweise mindestens 20 g bzw. weniger als 10, vorzugsweise weniger als 1 mg des Polymeren lösen.

Das Mischungsverhältnis der Polymeren a):b) soll im Bereich von 5:95 bis 95:5, vorzugsweise 10:90 bis 90:10 Gewichtsteilen liegen. Es ist erstaunlicherweise möglich, mit 6 % der Komponente a) Tabletten herzustellen, deren äußere Form (wenn auch sehr porös, quasi nur noch als Gerüst) im Verdauungstrakt unbeschädigt erhalten bleibt.

Die Komponente c) kann aus einem oder mehreren der üblichen Pharmahilfsstoffe bestehen. Als solche kommen beispielsweise in Betracht: Streck- und/oder Gleitmittel wie Silikate oder Kieselerde, Stearinsäure oder deren Salze mit z.B. Magnesium oder Kalzium, Polyethylenglykole, Cellulosederivate, Talkum, Saccharose, Lactose, Getreide- oder Maisstärke, Kartoffelmehl, ferner Netz-, Konservierungsmittel, Redoxstabilisatoren, Weichmacher, Adsorbentien, Geschmackstoffe (vgl. z.B. H. Sucker et al., Pharmazeutische Technologie, Thieme-Verlag, Stuttgart 1978).

Das Mischen des Wirkstoffs oder der Wirkstoffe mit den polymeren Bindemitteln und gegebenenfalls weiteren üblichen galenischen Zusätzen kann vor oder nach dem Schmelzen des polymeren Bindemittels nach den in der Technik üblichen Verfahren erfolgen. Bevorzugt wird das Mischen im Extruder, vorzugsweise einem Zweischneckenextruder oder einem Einschneckenextruder mit Mischabteil.

Das polymere Bindemittel sollte in der Gesamtmischung aller Komponenten im Bereich von 50 bis 180, vorzugsweise 60 bis 150°C erweichen oder schmelzen, so daß die Masse extrudierbar ist. Die Glasübergangstemperatur der Polymeren muß also auf jeden Fall unter 180°C liegen.

Die Schmelzen sind lösungsmittelfrei. Damit ist gemeint, daß kein Wasser und kein organisches Lösungsmittel zugesetzt wird.

Die Formgebung erfolgt durch Extrusion bei 50 bis 180°C, vorzugsweise 60 bis 150°C und anschließende kontinuierliche Verformung des noch plastischen Stranges, z.B. durch Verformen zu Tabletten, beispielsweise gemäß EP-A 240 906 durch Hindurchführen des Stranges zwischen zwei gegenläufig angetriebenen Walzen mit einander gegenüberliegenden Vertiefungen im Walzenmantel, deren Ausführung die Tablettenform bestimmt. Auch Kaltabschlag kommt in Betracht.

Bevorzugt wird der sogenannte Heißabschlag. Dabei werden die Stränge unmittelbar nach dem Austritt aus der Düsenanordnung am Extruder durch rotierende Messer zerkleinert, zweckmäßig in Stücke, deren Länge etwa gleich dem Strangdurchmesser ist. Diese abgeschlagenen Schmelzteilchen kühlen im Luft- oder Gasstrom so weit ab, daß die Oberfläche vor einer Berührung mit anderen Teilchen oder einer Gefäßwand bereits klebfrei ist, andererseits die Teilchen aber noch so plastisch sind, daß sie durch Zusammenstöße, z.B. mit der Wandung eines angeschlossenen Cyclons, eine sphärische Form erhalten. Man erhält so in einfacher Weise weitgehend kugel- oder linsenförmige Teilchen mit Durchmessern von 0,5 bis 4, vorzugsweise 0,8 bis 2 mm. Die bevorzugten kleineren Teilchen sind in erster Linie zum Füllen von Kapseln geeignet.

Falls gewünscht, kann die feste pharmazeutische Form auch mit einem üblichen Überzug zur Verbesserung des Aussehens und/oder des Geschmacks (Dragee) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt.

Die Erfindung gestattet in einfacher und umweltfreundlicher Weise (ohne Lösungsmittel) eine gezielte Retardeinstellung, weitgehend unabhängig von der Form und der Größe der Arzneiform. Die erfindungsgemäßen Arzneiformen bleiben im Verdauungstrakt weitgehend formstabil, so daß die Wirkstoffabgabe praktisch ausschließlich nach dem Diffusionsprinzip erfolgt. Die Erfindung eröffnet die Möglichkeit, auch eine pH-unabhängige Wirkstofffreisetzung zu erzielen. Der Retardeffekt kann auch bei kleinen Retardformen außerordentlich stark eingestellt werden. Die Streuung der Wirkstofffreisetzung ist aufgrund der guten Homogenität der Masse in Verbindung mit der Formstabilität gering und hervorragend reproduzierbar. Die Kinetik der Wirkstofffreisetzung bleibt auch unter extremen klimatischen Lagerbedingungen (Temperatur, Feuchtigkeit) überraschend stabil. Ferner ist es überraschend, daß die erfindungsgemäßen Arzneiformen im Gegensatz zu extrudierten Arzneiformen, die kein wasserunlösliches Polymeres enthalten, praktisch keinen kalten Fluß zeigen.

### Beispiele

### Beispiel 1

60 Teile Theophyllin, 10 Teile eines Copolymerisats vom K-Wert 30 aus 60 Gew.-% N-Vinylpyrrolidon und 40 Gew.-% Vinylacetat, 10 Teile Hydroxypropylcellulose (Klucel® EF, Fa. Aqualon), sowie 20 Teile eines Copolymerisats aus Ethylacrylat-Methylmethacrylat-Trimethylammonioethylmethacrylatchlorid (1:2:0,1 Molverhältnis; Eudragit® RS der Fa. Röhm Pharma, Weiterstadt) wurden in einem Doppelschneckenextruder vermischt und extrudiert. Die Temperatur der Schüsse des Extrudermantels betrug 70, 80, 100, 120°C, die Düse wurde auf 130°C aufgeheizt. Der erhaltene Schmelze-Strang wurde in einen Kalander mit 750 mg-Formprägungen eingeführt. Die Tabletten zeigten im No-Change-Test nach USP XXI bei pH = 1,2 und pH 6,8 eine vom pH-Wert sowie von der Paddle-Drehzahl unabhängige Wirkstofffreisetzung von 62 % in 8 h. Bei 50°C und 30°C/75 % relativer Luftfeuchtigkeit waren die Tabletten mindestens 1 Jahr lagerstabil.

### Beispiel 2

50 Teile Theophyllin, 30 Teile eines Copolymerisats vom K-Wert 30 aus 60 Gew.-% N-Vinylpyrrolidon und 40 Gew.-% Vinylacetat, sowie 20 Teile eines Copolymerisats aus Methylmethacrylat und Ethylacrylat (im Gewichtsverhältnis 2:1) wurden unter den Bedingungen des Beispiels 1 verarbeitet und mit 500 mg-Formen kalandriert. Die Wirkstofffreisetzung betrug nach 8 h pH-unabhängig 68 %.

### Beispiel 3

50 Teile Theophyllin, 10 Teile Hydroxypropylcellulose sowie 30 Teile eines Copolymerisats aus Ethylacrylat-Methylmethacrylat-Trimethylammonioethylmethacrylatchlorid (1:2:0,1 Molverhältnis) und 10 Teile eines Copolymerisats aus Ethylacrylat-Methylmethacrylat-Trimethylammonioethylmethacrylat-chlorid (1:2:0,2 Molverhältnis, Eudragit® RL der Fa. Röhm Pharma, Weiterstadt) wurden in einem Doppelschneckenextruder vermischt und extrudiert. Die Temperatur der Schüsse des Extrudermantels betrug 70, 80, 100, 120°C, die Düse wurde auf 130°C aufgeheizt. Die aus der Düsenanordnung mit 1,0 mm Düsendurchmesser austretenden Schmelzestränge wurden durch ein rotierendes Messer so abgeschlagen ("Heißabschlag"), daß nach dem Abkühlen im Luftstrom und dem Abscheiden im Cyclon weitgehend kugelförmige Teilchen erhalten wurden. 90 % der Teilchen hatten einen Durchmesser Zwischen 1,0 und 1,5 mm. Die Freisetzung des Wirkstoffs erreichte 60 % in 8 h.

### Beispiel 4

6 Teile Biperiden, 9 Teile eines Copolymerisats vom K-Wert 30 aus 60 Gew.-% N-Vinylpyrrolidon und 40 Gew.-% Vinylacetat, 10 Teile Hydroxypropylcellulose, sowie 75 Teile eines Copolymerisats aus Ethylacrylat-Methylmethacrylat-Trimethylammonioethylmethacrylatchlorid (1:2:0,1 Molverhältnis) wurden extrudiert (70, 80, 100, 110, Düsen 110°C) und heiß abgeschlagen (Düsendurchmesser 0,8 mm). 95 % der Pellets hatten einen Durchmesser zwischen 0,8 und 1,3 mm. Die Freisetzung des Wirkstoffs betrug 83 % in 8 h.

### Beispiel 5

Aus 20 Teilen Nifedipin, 62,5 Teilen eines Copolymerisates vom K-Wert 30 aus 60 Gew.-% N-Vinylpyrrolidon und 40 Gew.-% Vinylacetat sowie 17,5 Teilen eines Copolymerisats aus Ethylacrylat-Methylmethacrylat-Trimethylammonioethylmethacrylat-chlorid (1:2:0,1 Molverhältnis) wurden wie in den obengenannten Beispielen Pellets hergestellt (Temperatur der Schüsse 80/100/100/115°C, Düsentemperatur 120°C, Düsendurchmesser 1,0 mm). 87 % der Teilchen hatten einen Durchmesser zwischen 1,25 mm und 1,8 mm. Die Wirkstofffreisetzung erfolgte zu 52 % in 8 h. Sowohl in röntgenographischen Debye-Scherrer-Messungen als auch in DSC-Untersuchungen konnten keine kristallinen Nifedipin-Anteile nachgewiesen werden.

### Beispiel 6

40 Teile Melperon, 30 Teile eines Copolymerisats vom K-Wert 30 aus 60 Gew.-% N-Vinylpyrrolidon und 40 Gew.-% Vinylacetat, sowie 30 Teile eines Copolymerisats aus Ethylacrylat-Methylmethacrylat-Trimethylammonioethylmethacrylat-chlorid (1:2:0,1 Molverhältnis) wurden extrudiert (Temperatur der Schüsse: 60, 70, 80, 90, 100°C, Düsentemperatur 120°C) und mit 500 mg-Formwalzen kalandriert. Die Tabletten zeigten im No-Change-Test nach USP XXI bei pH = 1,2 und pH = 6,8 eine pH-unabhängige Wirkstofffreisetzung von 85 % in 8 h.

### Beispiel 7

40 Teile Melperon, 12 Teile eines Copolymerisats vom K-Wert 30 aus 60 Gew.-% N-Vinylpyrrolidon und 40 Gew.-% Vinylacetat, 8 Teile Hydroxypropylcellulose, sowie 40 Teile eines Copolymerisats aus Ethylacrylat-Methylmethacrylat-Trimethylammonioethylmethacrylat-chlorid (1:2:0,1 Molverhältnis) wurden extrudiert und heiß abgeschlagen (s. Beispiel 3, Temperatur der Schüsse 60, 80, 90, 110°C, Düsentemperatur 120°C, Düsendurchmesser 1,0 mm). 95 % der Pellets hatten einen Durchmesser zwischen 1,2 und 1,8 mm. Die Wirkstofffreisetzung betrug 96 % in 8 h.

## Patentansprüche

1. Feste pharmazeutische Retardform, hergestellt durch Schmelzextrusion bei 50 bis 200°C und kontinuerliche Formgebung einer Mischung von 0,1 bis 70 Gew.-%, bezogen auf die fertige Retardform, eines pharmazeutischen Wirkstoffs mit einer Polymerschmelze folgender Zusammensetzung:
a) mindestens 6 Gew.-%, bezogen auf die gesamte Retardform, mindestens eines wasserunlöslichen Poly(meth)acrylates mit einer Glastemperatur Tg im Bereich -60 bis 180°C,
b) eine wasserlösliche Hydroxyalkylcellulose oder Hydroxyalkylmethylcellulose mit 2 oder 3 C-Atomen im Hydroxyalkylrest oder ein N-Vinylpyrrolidonpolymerisat mit 0 bis 50 Gew.% Vinylacetat oder eine Mischung von beiden im Mischungsverhältnis a):b) = 5:95 bis 95:5, und
c) 0 bis 30 Gew.%, bezogen auf die fertige Retardform, eines oder mehrerer der üblichen Pharmahilfsstoffe.

2. Feste pharmazeutische Retardform gemäß Anspruch 1, dadurch gekennzeichnet, daß das Polymer a) aus einem Copolymerisat von Acrylaten mit C₁- bis C₈-Alkoholen oder Methacrylaten mit C₁- bis C₄-Alkoholen und 0 bis 20 Mol.% eines quaternäre Ammoniumgruppen enthaltenden (Meth)Acrylates besteht.

3. Feste pharmazeutische Retardform nach Anspruch 1 oder 2, dadurch gekennzeichnet, daß die Formgebung durch Heißabschlag erfolgt ist.

## Claims

1. A solid depot drug form produced by melt extrusion at from 50 to 200°C and continuous shaping of a mixture of from 0.1 to 70% by weight, based on the finished depot form, of a pharmaceutical active ingredient with a polymer melt of the following composition:
a) at least 6% by weight, based on the complete depot form, of at least one water-insoluble poly(meth)acrylate with a glass transition temperature Tg in the range from -60 to 180°C,
b) a water-soluble hydroxyalkylcellulose or hydroxyalkylmethylcellulose with 2 or 3 carbons in the hydroxyalkyl, or an N-vinylpyrrolidone polymer with from 0 to 50% by weight of vinyl acetate or a mixture of the two in the ratio a):b) = 5:95 to 95:5, and
c) 0-30% by weight, based on the finished depot form, of one or more conventional pharmaceutical auxiliaries.

2. A solid depot drug form as claimed in claim 1, wherein polymer a) is composed of a copolymer of acrylates with C₁-C₈-alcohols or methacrylates with C₁-C₄-alcohols and from 0 to 20 mol% of a (meth)acrylate containing quaternary ammonium groups.

3. A solid depot drug form as claimed in claim 1 or 2, wherein shaping is by hot-cut pelletization.

## Revendications

1. Forme pharmaceutique retard solide, préparée par extrusion à l'état fondu, à une température de 50 à 200°C, et façonnage continu d'un mélange de 0,1 à 70% en poids, par rapport à la forme retard prête à l'emploi, d'une substance pharmaceutique active avec une masse fondue de polymère ayant la composition suivante:
a) au moins 6% en poids, par rapport à la forme retard totale, d'au moins un poly(méth)acrylate insoluble dans l'eau, ayant une température de transition vitreuse comprise entre -60 et 180°C,
b) une hydroxyalkylcellulose ou une hydroxyalkylméthylcellulose soluble dans l'eau, contenant 2 ou 3 atomes de carbone dans le reste hydroxyalkyle, un polymère de N-vinylpyrrolidone avec 0 à 50% en poids d'acétate de vinyle ou un mélange des deux, dans un rapport de mélange a) : b) = 5 : 95 à 95 : 5, et
c) 0 à 30% en poids, par rapport à la forme retard prête à l'emploi, d'un ou de plusieurs des produits pharmaceutiques auxiliaires usuels.

2. Forme pharmaceutique retard solide selon la revendication 1, caractérisée en ce que le polymère a) est constitué par un copolymère d'acrylates avec des alcools en C₁ à C₈ ou de méthacrylates avec des alcools en C₁ à C₄ et de 0 à 20% en moles d'un (méth)acrylate contenant des groupements ammonium quaternaires.

3. Forme pharmaceutique retard solide selon la revendication 1 ou 2, caractérisée en ce que le façonnage est effectué par découpage à chaud.
